# EUROPÄISCHE PATENTSCHRIFT

(11) **EP 3 571 975 B1**
(45) Veröffentlichungstag und Bekanntmachung des Hinweises auf die Patenterteilung: **02.07.2025**
(21) Anmeldenummer: 19172294.1
(22) Anmeldetag: 02.05.2019
(51) Int. Cl.: A61B 1/00, G02B 23/24

(54) **MEDIZINISCHES ENDOSKOP MIT EINER ABBILDUNGSEINRICHTUNG**
MEDICAL ENDOSCOPE COMPRISING AN IMAGING DEVICE
ENDOSCOPE MÉDICAL AVEC UN DISPOSITIF D'IMAGERIE

(30) Priorität: 02.05.2018 DE 102018110523
(43) Veröffentlichungstag der Anmeldung: 27.11.2019
(73) Patentinhaber: Universität Stuttgart, 70174 Stuttgart (DE); Karl Storz SE & Co. KG, 78532 Tuttlingen (DE)
(72) Erfinder: Gießen, Harald, 67297 Marnheim (DE); Herkommer, Alois, 70569 Stuttgart (DE); Thiele, Simon, 70569 Stuttgart (DE); Irion, Klaus, 78532 Tuttlingen (DE); Göbel, Werner, 78532 Tuttlingen (DE); Häsler, Benjamin, 78532 Tuttlingen (DE)
(74) Vertreter: Wimmer, Stephan

(56) Entgegenhaltungen:
- EP-A1- 0 647 425
- EP-A1- 2 037 307
- EP-A1- 2 056 150
- EP-A1- 3 162 549
- WO-A1-2017/059960
- WO-A2-2009/019703
- US-A1- 2005 272 979
- CHEN XIANGFAN ET AL: "High-Speed 3D Printing of Millimeter-Size Customized Aspheric Imaging Lenses with Sub 7 nm Surface Roughness", ADVANCED MATERIALS, vol. 30, no. 18, 24 March 2018 (2018-03-24), DE, pages 1705683, XP055941744, ISSN: 0935-9648, DOI: 10.1002/adma.201705683

## Beschreibung

Die vorliegende Erfindung ist auf ein medizinisches Endoskop mit einer optischen Abbildungseinrichtung zum Erzeugen eines reellen Bilds eines mittels des Endoskops betrachteten Objekts bezogen.

In vielen medizinischen Teilgebieten der Medizin ist eine optische Inspektion dünnkalibriger Hohlräume oder Hohlorgane diagnostisch vorteilhaft und wünschenswert. Beispiele sind Zahnwurzelkänale mit einem typischen Durchmesser von 0,5 mm oder weniger, Speichelgänge mit einem typischen Durchmesser von 1 mm oder weniger, die Eustachi-Röhren mit einem typischen Durchmesser von 1,5 mm oder weniger und Milchgänge mit einem typischen Durchmesser von 1,3 mm oder weniger. Ferner könnte zukünftig - bei geeigneter Spülung - eine optische Inspektion der Innenwände von Blutgefäßen oder von Lymphgefäßen diagnostisch vorteilhaft und wünschenswert sein. Im Rahmen der Bronchoskopie wäre eine optische Inspektion von Alveolen diagnostisch vorteilhaft und wünschenswert. Auch eine optische Inspektion eines Embryos im Mutterleib wäre diagnostisch vorteilhaft und wünschenswert. Diese Anwendungen erfordern entsprechend dünne optische Inspektionsinstrumente, insbesondere Endoskope.

Neue Fertigungsverfahren ermöglichen eine immer präzisere Fertigung immer kleinerer Strukturen, auch aus optisch transparenten Materialien.

In EP 1 961 371 B1 ist eine Beleuchtungseinrichtung für eine Bilderfassungseinrichtung am distalen Ende eines Endoskops beschrieben. Zur Strahlformung sind Mikrolinsen vorgesehen (Absatz [0024]).

In DE 10 2015 003 652 A1 sind ein Verfahren zum Verbinden einer optischen Festkernfaser mit einer weiteren optischen Faser und ein Verfahren zum Bereitstellen einer optischen Festkernfaser mit einer Fügungsvorrichtung zum Verbinden der optischen Festkernfaser mit einer weiteren optischen Faser beschrieben (Titel, Absätze [0015], [0016]). Die Fügungsvorrichtung wird mittels 3D-Drucks basierend auf Zwei-Photonen-Polymerisation auf das axiale Ende der Festkernfaser aufgebracht (Absätze [0016], [0021]).

In DE 10 2015 012 980 A1 sind ein Verfahren und eine Vorrichtung zur Herstellung von Mikrostrukturen auf optischen Fasern mittels eines 3D-Druckers beschrieben (Titel, Absätze [0007], [0009], [0058], [0064], [0067]). Dabei kann Zwei-Photonen-Polymerisation verwendet werden (Absatz [0059]).

In EP 3 162 549 A1 sind ein Verfahren und eine Vorrichtung zur Herstellung eines optischen Elements mit einer Blende oder einer Struktur zur Streulichtabsorption beschrieben (Titel, Absätze [0004], [0008], [0012]). Mittels eines 3D-Druckers wird eine dreidimensionale Struktur mit einer mikrofluidischen Kavität gebildet, die mikrofluidische Kavität wird anschließend durch Kapillareffekt mit einer funktionalen Substanz gefüllt (Absatz [0015]).

In DE 10 2015 103 214 A1 ein Trokar 20 beschrieben, der ein Durchdringen der Bauchdecke unter optischer Kontrolle ermöglichen soll. Dazu weist der Trokar einen Hohlraum, in dem ein Schaft eines Endoskops angeordnet werden kann, und eine zumindest teilweise optisch transparente Spitze 24 auf. Zwischen der vorgesehenen Position des distalen Endes 11 des Endoskops 10 und der transparenten Spitze 24 des Trokars 20 sind Linsen 30 und Prismen 40 vorgesehen, um die Blickrichtung von der Endoskop-Blickrichtung zu einer Trokar-Blickrichtung zu ändern und mit einem Seitenblick-Endoskop einen Geradeausblick im Trokar zu ermöglichen.

In DE 697 34 638 T2 ist ein optisches System eines Endoskops mit einer rotationsasymmetrischen gewölbten Fläche beschrieben.

In US 5,912,764 ist ein optisches System für ein Endoskop beschrieben (Spalte 1, Zeilen 5 bis 8; Spalte 1, Zeile 66, bis Spalte 2, Zeile 1).

In DE 10 2011 089 157 A1 ist ein Videoendoskop mit seitlicher Blickrichtung beschrieben (Absätze [0001], [0007]). Das Videoendoskop 1 umfasst am distalen Ende seines Endoskopschafts 2 eine Meniskus-Linse 12, ein Prisma 11, das seitlich einfallende Lichtstrahlen in eine axiale Richtung umlenkt, und Linsen 23, 23' vor einem optischen Sensor 21 (Absatz [0029], Figur).

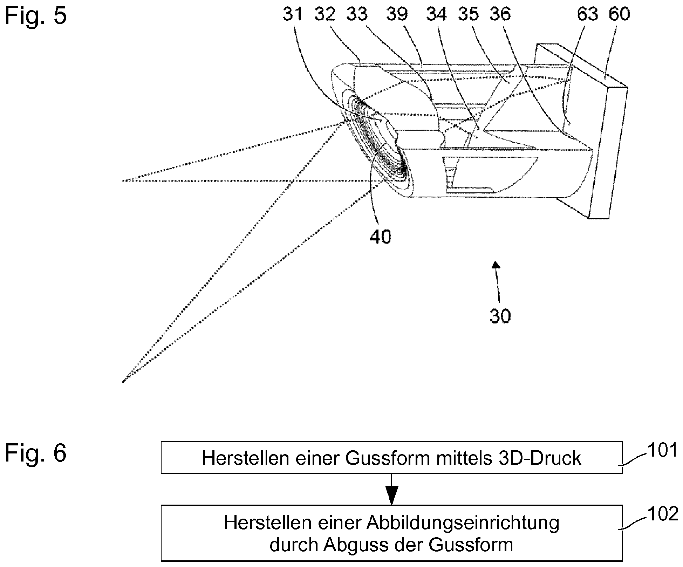

In WO 2009/019703 A2 ist eine Laryngoskopvorrichtung beschrieben (Titel; Zusammenfassung; Seite 1, Zeilen 4, 5). Eine Aperturlinse ("aperture lens") 66 weist eine proximale konkave Oberfläche 68 und eine distale konkave, gekippte Prismaoberfläche 69 auf (Seite 6, Zeilen 22 bis 24; Figur 5).

In EP 0 647 425 A1 ist eine Endoskop-Ergänzung ("endoscope attachment") zur Veränderung des Blickwinkels beschrieben (Titel; Seite 2, Zeilen 5, 6). In Lichtausbreitungsrichtung vor einem Prisma 24, 50, 66, in dem Totalreflexion stattfindet, ist eine plankonkave Zerstreuungslinse 26, 56 angeordnet, nach dem Prisma 24, 50, 66 ist eine plankonvexe Sammellinse 58 angeordnet, die offensichtlich ein reelles Bild erzeugen soll.

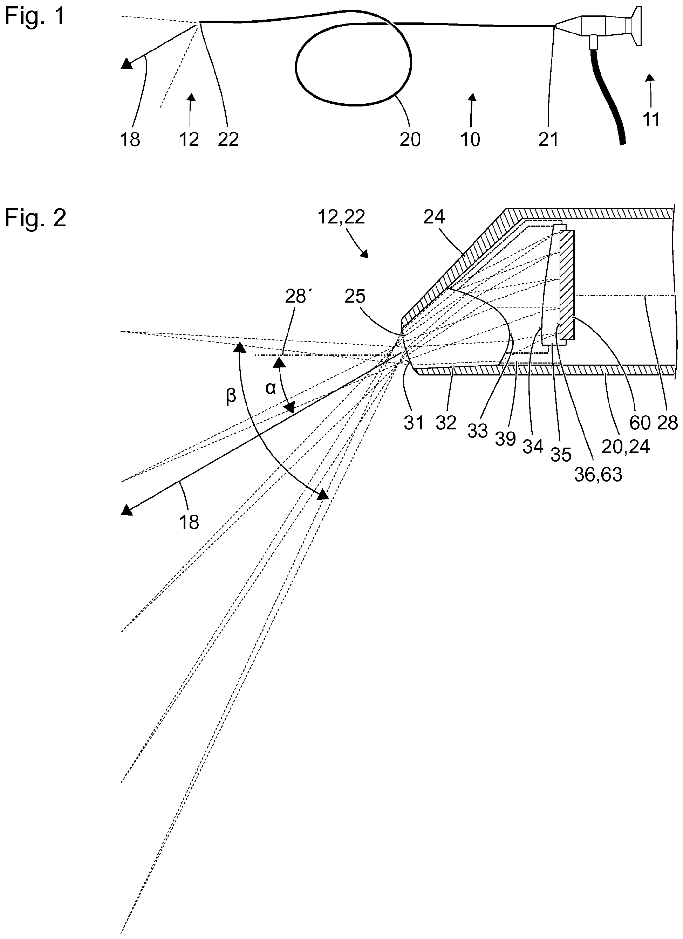

In EP 2 056 150 A1 ist ein übertragendes optisches Element ("transmissive optical element") 2 beschrieben (Titel, Zusammenfassung, Absätze [0001], [0075], Figuren 1, 4, 5, 8, 9, 10, 13, 16, 19, 22). Das übertragende optische Element ist rotationssymmetrisch (Absätze [0005], [0006], [0011], [0020], [0021], [0045] etc.).

In dem Artikel "High-Speed 3D Printing of Millimeter-Size Customized Aspheric Imaging Lenses with Sub 7 nm Surface Roughness" (Xiangfan Chen et al., Advanced Materials, Band 30, Ausgabe 18, 2018) ist ein Verfahren zum Herstellen von kundenspezifischen optischen Elementen beschrieben. Mittels einer dynamischen Maske kann bei einer einzigen Belichtung anstelle eines einzelnen Voxels eine gesamte zweidimensionale Schicht photopolymerisiert werden.

In US 2005/0272979 A1 ist eine visuelle Einrichtung eines Endoskops beschrieben. Die visuelle Einrichtung umfasst eine Optik oder einen Optikverbund aus mehreren Optiken und realisiert einen Öffnungswinkel von mehr als 90 Grad.

Eine Aufgabe der vorliegenden Erfindung besteht darin, ein verbessertes Endoskop zu schaffen.

Diese Aufgabe wird durch den Gegenstand des unabhängigen Anspruchs 1 gelöst.

Weiterbildungen sind in den abhängigen Ansprüchen angegeben.

Ein Endoskop umfasst einen Schaft mit einem distalen Ende, eine optische Abbildungseinrichtung an dem distalen Ende des Schafts zum Erzeugen eines reellen Bilds eines mittels des Endoskops betrachteten Objekts und zumindest entweder eine Bildübertragungseinrichtung zum Weiterleiten des reellen Bilds oder einen Bildsensor zum Erfassen des reellen Bilds, wobei die Abbildungseinrichtung gekrümmte lichtbrechende Grenzflächen aufweist, die gegeneinander gekippt sind, wobei die Blickrichtung des medizinischen Endoskops nicht parallel zu der Längsachse des distalen Endes des Schafts des medizinischen Endoskops ist, und wobei die Abbildungseinrichtung mittels 3D-Druck hergestellt ist.

Das Endoskop ist erfindungsgemäß für medizinische Anwendungen vorgesehen, beispielsweise als Sialendoskop für die optische Inspektion eines Speichelgangs, als Fetoskop zur optischen Inspektion eines ungeborenen Fötus in der Fruchtblase, als Zystoskop zur Blasenspiegelung, als Wurzelkanal-Endoskop zur optischen Inspektion des Wurzelkanals eines Zahns in der Endodontie, oder zur Duktoskopie, d. h. zur optischen Inspektion eines Milchkanals einer Brustdrüse. Alternativ, und nicht von dem Gegenstand der Ansprüche umfasst, kann das Endoskop für technische Anwendungen vorgesehen und ausgebildet sein.

Der Schaft des Endoskops ist insbesondere lang und dünn. Der Schaft kann gerade oder gekrümmt, starr oder flexibel sein.

Die Bildübertragungseinrichtung kann beispielsweise eine Mehrzahl hintereinander angeordneter Stablinsen oder ein anderes Relaislinsensystem oder ein geordnetes Bündel von Lichtleitfasern aufweisen. Die Bildübertragungseinrichtung überträgt das von der optischen Abbildungseinrichtung erzeugte reelle Bild insbesondere zu einem proximalen Ende des Endoskops. An dem proximalen Ende des Endoskops kann das übertragene reelle Bild durch ein Okular unmittelbar beobachtet oder von einer an das Okular gekoppelten Kamera oder von einem oder mehreren auf andere Weise mit dem Endoskop optisch gekoppelten oder in das Endoskop integrierten Bildsensoren erfasst und in ein Bildsignal gewandelt werden. Alternativ ist ein Bildsensor oder sind mehrere Bildsensoren unmittelbar an der Abbildungseinrichtung angeordnet, um das von der Abbildungseinrichtung erzeugte reelle Bild zu erfassen und in ein analoges oder digitales Bildsignal zu wandeln.

Die gekrümmten lichtbrechenden Grenzflächen sind insbesondere so gegeneinander gekippt, dass es keine gemeinsame Symmetrieachse gibt, zu der alle gekrümmten lichtbrechenden Grenzflächen rotationssymmetrisch wären. Insbesondere sind die Flächennormalen der gekrümmten lichtbrechenden Grenzflächen an deren Scheiteln oder an deren Flächenmittelpunkten nicht parallel zueinander und/oder nicht parallel zu einer Verbindungsgeraden der Scheitel oder Flächenmittelpunkte. Alternativ können beispielsweise die mittleren Flächennormalen der gekrümmten lichtbrechenden Grenzflächen nicht parallel zueinander und/oder nicht parallel zu einer Verbindungsgeraden der Scheitel oder Flächenmittelpunkte der gekrümmten lichtbrechenden Grenzflächen sein. Diese Anordnung und Ausrichtung der gekrümmten lichtbrechenden Grenzflächen wird auch als außeraxial bezeichnet. Die Abbildungseinrichtung kann außer zwei oder mehr gekrümmten lichtbrechenden Grenzflächen die gegeneinander gekippt sind, auch eine oder mehrere weitere gekrümmte lichtbrechende Grenzflächen aufweisen, die zueinander oder zu einer der gegeneinander gekippten gekrümmten lichtbrechenden Grenzflächen parallel oder symmetrisch zu einer gemeinsamen optischen Achse angeordnet sind.

Eine Abbildungseinrichtung mit gekrümmten lichtbrechenden Grenzflächen, die gegeneinander gekippt sind, ermöglicht ungewöhnliche Abbildungseigenschaften, beispielsweise ein Abweichen der Blickrichtung von der Flächennormalen des durch die optische Abbildungseinrichtung erzeugten reellen Bilds, eine Verzerrung oder Entzerrung und/oder eine Nicht-Parallelität von Gegenstandsfläche und Bildfläche.

Bei einem Endoskop, wie es hier beschrieben ist, ist der Durchmesser des Schafts insbesondere nicht größer als 3 mm oder nicht größer als 1,5 mm oder nicht größer als 1,0 mm oder kleiner als 1,0 mm.

Bei einem Endoskop, wie es hier beschrieben ist, ist der Durchmesser der Abbildungseinrichtung insbesondere nicht größer als 3 mm oder nicht größer als 1,5 mm oder nicht größer als 1,0 mm oder kleiner als 1,0 mm.

Bei einem Endoskop, wie es hier beschrieben ist, ist insbesondere eine der gekrümmten lichtbrechenden Grenzflächen der Abbildungseinrichtung nicht rotationssymmetrisch.

Eine gekrümmte lichtbrechende Grenzfläche ist nicht rotationssymmetrisch, wenn es keine Symmetrieachse gibt, zu der die gekrümmte lichtbrechende Grenzfläche rotationssymmetrisch wäre. Die Abbildungseinrichtung kann mehrere gekrümmte lichtbrechende Grenzflächen aufweisen, die jeweils nicht rotationssymmetrisch sind.

Bei einem Endoskop, wie es hier beschrieben ist, ist insbesondere eine der gekrümmten lichtbrechenden Grenzflächen der Abbildungseinrichtung kein Ausschnitt aus einer rotationssymmetrischen gekrümmten Fläche.

Insbesondere sind mehrere oder alle gekrümmten lichtbrechenden Grenzflächen der Abbildungseinrichtung nicht Ausschnitte aus rotationssymmetrischen gekrümmten Flächen.

Bei einem Endoskop, wie es hier beschrieben ist, ist insbesondere eine der gekrümmten lichtbrechenden Grenzflächen eine Freiformfläche.

Insbesondere können mehrere oder alle gekrümmten lichtbrechenden Grenzflächen Freiformflächen sein. Als Freiformfläche wird insbesondere eine Fläche bezeichnet, die nicht ein Ausschnitt einer Kugeloberfläche oder eines Rotationsellipsoids oder eines Rotationshyperboloids ist. Die Ausgestaltung von einer oder mehreren gekrümmten lichtbrechenden Grenzflächen der Abbildungseinrichtung als Freiflächen kann eine weitgehende Anpassung der optischen Eigenschaften der Abbildungseinrichtung an Erfordernisse der vorgesehenen Anwendung und gleichzeitig eine hohe Abbildungsqualität ermöglichen.

Bei einem Endoskop, wie es hier beschrieben ist, ist die Blickrichtung des Endoskops erfindungsgemäß nicht parallel zu der Längsachse des distalen Endes des Schafts des Endoskops.

Die Blickrichtung des Endoskops ist insbesondere die auf die Mitte der Lichteintrittsfläche des Endoskops bezogene Richtung, in der ein Objekt liegt, das in die Mitte eines mittels des Endoskops erfassten Bilds abgebildet wird. Insbesondere im Fall einer verzerrten Abbildung mit an unterschiedlichen Orten des Bilds deutlich unterschiedlichen Abbildungsmaßstäben kann als Blickrichtung des Endoskops angesehen werden die Richtung von der Mitte der Lichteintrittsfläche des Endoskops zu dem Flächenmittelpunkt jener Fläche, die von der Abbildungseinrichtung scharf abgebildet und vollständig von der Bildübertragungseinrichtung weitergeleitet oder von dem Bildsensor erfasst wird.

Endoskope, deren Blickrichtung nicht parallel zu der Längsachse des Schafts (im Fall eines gekrümmten oder krümmbaren Schafts: nicht parallel zu der Längsachse des Schafts an seinem distalen Ende) ist, werden oft auch als Seitenblick-Endoskope bezeichnet. Gekrümmte lichtbrechende Grenzflächen, die gegeneinander gekippt sind, können eine von der Längsachse des Schafts abweichende Blickrichtung des Endoskops ermöglichen, ohne dabei reflektierende Flächen (beispielsweise an einem Prisma) oder eine schräge Anordnung des Bildsensors zu erfordern.

Bei einem Endoskop, wie es hier beschrieben ist, ist der Winkel zwischen der Blickrichtung des Endoskops und der Längsachse des distalen Endes des Endoskops insbesondere nicht kleiner als 10 Grad oder nicht kleiner als 20 Grad oder nicht kleiner als 30 Grad.

Bei einem Endoskop, wie es hier beschrieben ist, beträgt ein Winkel zwischen der Blickrichtung des Endoskops und der Längsachse des distalen Endes des Endoskops insbesondere 25 Grad oder 30 Grad oder 45 Grad oder 60 Grad oder 70 Grad oder 90 Grad oder 120 Grad.

Bei einem Endoskop, wie es hier beschrieben ist, wird zur Erzeugung des reellen Bilds beitragendes Licht insbesondere nicht reflektiert.

Bei einem Endoskop, wie es hier beschrieben ist, ist insbesondere keine reflektierende Fläche lichtstromaufwärts des von der Abbildungseinrichtung erzeugten reellen Bilds vorgesehen.

Bei einem Endoskop, wie es hier beschrieben ist, weist insbesondere die Abbildungseinrichtung keine reflektierende Fläche auf.

Der Verzicht auf eine reflektierende Fläche kann den erforderlichen Bauraum verringern und eine präzise Fertigung vereinfachen. Insbesondere muss kein Prisma mit totalreflektierenden Oberflächenbereichen gefertigt und angeordnet werden.

Bei einem Endoskop, wie es hier beschrieben ist, ist der Bildwinkel des Endoskops insbesondere nicht kleiner als 60 Grad oder nicht kleiner als 70 Grad oder nicht kleiner als 80 Grad oder nicht kleiner als 90 Grad oder nicht kleiner als 100 Grad.

Bildwinkel eines Endoskops ist insbesondere der von der Mitte der Lichteintrittsfläche des Endoskops aus gemessene Winkel zwischen einander gegenüberliegenden geraden oder im Wesentlichen geraden Randabschnitten derjenigen Fläche, die von der Abbildungseinrichtung scharf abgebildet und deren reelles Bild von der Bildübertragungseinrichtung vollständig weitergeleitet oder von dem Bildsensor vollständig erfasst wird. Alternativ ist der Bildwinkel eines Endoskops insbesondere der von der Mitte der Lichteintrittsfläche des Endoskops aus gemessene Winkel zwischen einander gegenüberliegenden Randabschnitten derjenigen Fläche, die von der Abbildungseinrichtung scharf abgebildet und deren reelles Bild von der Bildübertragungseinrichtung vollständig weitergeleitet oder von dem Bildsensor vollständig erfasst wird.

Gekrümmte lichtbrechende Grenzflächen, die gegeneinander gekippt sind, können selbst bei einer Blickrichtung, die nicht parallel zu der Längsachse des distalen Endes des Schafts des Endoskops ist, einen großen Bildwinkel und gleichzeitig eine vergleichsweise gute Abbildungsqualität ermöglichen.

Bei einem Endoskop, wie es hier beschrieben ist, weist die Abbildungseinrichtung zumindest entweder Kunststoff oder Glas oder eine Mischung aus Kunststoff und Glas auf oder ist teilweise oder vollständig aus Kunststoff oder Glas oder einer Mischung aus Kunststoff und Glas gebildet.

Glas kann in Form von Nanopartikeln oder anderen kleinen Partikeln in eine Kunststoffmatrix eingebettet sein. Unterschiedliche optische Eigenschaften, beispielsweise gegensätzliche Dispersion des Glases der Glaspartikel und des Kunststoffs können beispielsweise eine Minderung der chromatischen Aberration ermöglichen.

Ein Endoskop, wie es hier beschrieben ist, umfasst insbesondere ferner eine Blendenstruktur zum lateralen Beschneiden eines optischen Pfads, wobei die Blendenstruktur zumindest entweder gleichzeitig mit der Abbildungseinrichtung hergestellt oder in die Abbildungseinrichtung eingebettet ist.

Die Blendenstruktur ist insbesondere aus einem lichtabsorbierenden Material gebildet. Die Blendenstruktur kann an einer lichtbrechenden Grenzfläche (und damit diese lateral begrenzend), zwischen zwei lichtbrechenden Grenzflächen oder, bezogen auf die Ausbreitungsrichtung von Licht, das von einem betrachteten Objekt ausgeht, vor oder hinter allen lichtbrechenden Grenzflächen der Abbildungseinrichtung angeordnet sein. Die Blendenstruktur kann beispielsweise mittels 3D-Druck oder durch Auffüllen eines Hohlraums in einem transparenten Körper der Abbildungseinrichtung durch Kapillarkraft gebildet sein.

Bei einem Endoskop, wie es hier beschrieben ist, ist die Abbildungseinrichtung insbesondere mittels auf Mehrphotonen-Absorption (insbesondere Zwei-Photonen-Absorption) oder Mehrphotonen-Polymerisation (insbesondere Zwei-Photonen-Polymerisation) beruhendem 3D-Druck hergestellt.

Bei einem Endoskop, wie es hier beschrieben ist, ist die Abbildungseinrichtung erfindungsgemäß dere mittels 3D-Druck hergestellt, wobei die Abbildungseinrichtung unmittelbar auf eine distale Lichteintrittsfläche einer Bildübertragungseinrichtung zum Weiterleiten des reellen Bild oder auf eine Lichteintrittsfläche eines Bildsensors zum Erfassen des reellen Bilds gedruckt ist.

Bei einem Endoskop, wie es hier beschrieben ist, ist die Abbildungseinrichtung insbesondere mittels 3D-Druck aus mehreren verschiedenen Materialien hergestellt.

Die Abbildungseinrichtung ist insbesondere aus mehreren verschiedenen Materialien mit unterschiedlichen Brechungsindizes gebildet. Die Abbildungseinrichtung ist insbesondere aus mehreren verschiedenen flüssigen photoreaktiven oder photohärtenden Vorläufermaterialen hergestellt.

Bei einem Endoskop, wie es hier beschrieben ist, weist die Abbildungseinrichtung insbesondere eine als diffraktives optisches Element ausgebildete Grenzfläche oder ein anderes diffraktives optisches Element auf.

Das diffraktive optische Element kann insbesondere eine chromatische Korrektur ermöglichen, indem es eine zu dem optisch transparenten Material der Abbildungseinrichtung entgegengesetzte Dispersion aufweist.

Bei einem Endoskop, wie es hier beschrieben ist, weist die Abbildungseinrichtung insbesondere mehrere optisch transparente und bei der vorgesehenen Verwendung von Licht, das von einem betrachteten Objekt ausgeht und zur Erzeugung des reellen Bild beiträgt, durchsetzte Körper mit den lichtbrechenden Grenzflächen auf.

Bei einem Endoskop, wie es hier beschrieben ist, weist die Abbildungseinrichtung insbesondere mehrere optisch transparente und bei der vorgesehenen Verwendung von Licht, das von einem betrachteten Objekt ausgeht und zur Erzeugung des reellen Bild beiträgt, durchsetzte Körper mit den lichtbrechenden Grenzflächen auf, wobei eine Stützeinrichtung die Körper mechanisch starr verbindet.

Die mehreren optisch transparenten Körper sind insbesondere so angeordnet, dass Licht, das von einem betrachteten Objekt ausgeht zu der Erzeugung des reellen Bilds beiträgt, die transparenten Körper nacheinander durchtritt.

Die Stützeinrichtung oder Stützstruktur umfasst beispielsweise eine oder mehrere Stützstreben. Die Stützeinrichtung oder Stützstruktur ist insbesondere verbunden mit Randbereichen der Körper mit den lichtbrechenden Grenzflächen. Die Stützeinrichtung kann mehrere, optional parallele Stützstreben und/oder ein Netz oder Gitter aus Stützstreben aufweisen.

Bei einem Endoskop, wie es hier beschrieben ist, sind die Stützeinrichtungen und die transparenten Körper, die von dem Licht durchsetzt werden und die gekrümmten lichtbrechenden Grenzflächen aufweisen insbesondere zumindest entweder aus dem gleichen Material gebildet oder gleichzeitig hergestellt.

Eine gleichzeitige Herstellung der Stützeinrichtung und der von dem Licht durchsetzten und die gekrümmten lichtbrechenden Grenzflächen aufweisenden Körper kann eine präzise relative Anordnung der gekrümmten lichtbrechenden Grenzflächen ermöglichen und damit eine Justage obsolet machen und die Fertigungskosten deutlich reduzieren.

Ein Endoskop, wie es hier beschrieben ist, umfasst erfindungsgemäß insbesondere ferner eine Lichtlenkeinrichtung an dem distalen Ende des Endoskops zum Lenken von aus einer distalen Lichtaustrittsfläche eines Lichtleiters oder aus einer Lichtaustrittsfläche einer Lichtquelle austretendem Beleuchtungslicht, wobei die Lichtlenkeinrichtung eine lichtbrechende Grenzfläche, die gegenüber der Längsachse des distalen Endes des Lichtleiters oder gegenüber einer Flächennormalen der Lichtaustrittsfläche der Lichtquelle gekippt ist, aufweist. Das Endoskop umfasst insbesondere einen Lichtleiter in Gestalt einer oder mehrerer Lichtleitfasern zum Übertragen von Beleuchtungslicht zu dem distalen Ende des Endoskops. Die Lichtlenkeinrichtung kann auf eine Lichtaustrittsfläche des Lichtleiters oder der Lichtquelle unmittelbar aufgedruckt sein.

Die Lichtlenkeinrichtung kann mehrere lichtbrechende Grenzflächen aufweisen, durch die bei der vorgesehenen Verwendung Beleuchtungslicht tritt, das danach Objekte innerhalb des Sichtfelds beleuchtet. Die lichtbrechenden Grenzflächen der Lichtlenkeinrichtung sind insbesondere so angeordnet, dass Beleuchtungslicht nacheinander durch die lichtbrechenden Grenzflächen tritt. Die lichtbrechenden Grenzflächen sind also insbesondere bezogen auf die Ausbreitungsrichtung des Beleuchtungslichts hintereinander und nicht nebeneinander angeordnet.

Eine lichtbrechende Grenzfläche der Lichtlenkeinrichtung ist insbesondere insofern gegenüber der Längsachse des distalen Endes des Lichtleiters oder gegenüber der Flächennormale der Lichtaustrittsfläche der Lichtquelle gekippt, als die mittlere Flächennormale oder die Flächennormale am Flächenmittelpunkt der lichtbrechenden Grenzfläche nicht parallel zu der Längsachse des distalen Endes des Lichtleiters oder zu der Flächennormale der Lichtaustrittsfläche der Lichtquelle ist.

Die gekippte Anordnung von einer oder mehreren lichtbrechenden Grenzflächen der Lichtlenkeinrichtung kann ein Krümmen des distalen Endes des Lichtleiters oder ein Kippen der Lichtquelle ersetzen oder obsolet machen.

Bei einem Endoskop, wie es hier beschrieben ist, ist die lichtbrechende Grenzfläche der Lichtlenkeinrichtung insbesondere nicht rotationssymmetrisch.

Bei einem Endoskop, wie es hier beschrieben ist, ist die lichtbrechende Grenzfläche der Lichtlenkeinrichtung insbesondere kein Ausschnitt einer rotationssymmetrischen gekrümmten Fläche.

Ein Endoskop, wie es hier beschrieben ist, umfasst insbesondere ferner ein Stützgerüst, das die Abbildungseinrichtung und die Lichtlenkeinrichtung miteinander starr verbindet.

Bei einem Endoskop, wie es hier beschrieben ist, sind die Abbildungseinrichtung und die Lichtlenkeinrichtung insbesondere zumindest entweder miteinander mechanisch starr verbunden oder weisen das gleiche optisch transparente Material auf oder sind erfindungsgemäß monolithisch ausgebildet oder sind gleichzeitig hergestellt.

Insbesondere die gleichzeitige Herstellung (beispielsweise im Rahmen desselben 3D-Druckprozesses) aus dem gleichen optisch transparenten Material kann eine präzise und doch kostengünstige Fertigung ermöglichen.

Bei einem Endoskop, wie es hier beschrieben ist, weist die Lichtlenkeinrichtung insbesondere eine Ausnehmung zur Aufnahme des distalen Endes eines Lichtleiters auf, wobei die Ausnehmung die Position und die Orientierung des distalen Endes des Lichtleiters definiert.

Die Ausnehmung ist insbesondere nach proximal offen. Die Ausnehmung weist insbesondere die Gestalt eines Sacklochs, dessen Ende die Position der Lichtaustrittsfläche des Lichtleiters definiert, auf. Die Gestalt der Ausnehmung ist insbesondere so gewählt, dass der Lichtleiter in lateraler Richtung formschlüssig und mit geringem Spiel gehalten ist.

Ein Verfahren, das nicht Gegenstand der Ansprüche ist, zum Herstellen einer Abbildungseinrichtung für ein Endoskop umfasst insbesondere einen Schritt des Herstellens der Abbildungseinrichtung mittels 3D-Druck.

Ein Verfahren, das nicht Gegenstand der Ansprüche ist, zum Herstellen einer Abbildungseinrichtung für ein Endoskop umfasst insbesondere einen Schritt des Herstellens einer Gussform für die Abbildungseinrichtung und einen Schritt eines Herstellens der Abbildungseinrichtung durch Abguss der Gussform, wobei die Gussform mittels 3D-Druck hergestellt wird.

Die hier beschriebenen Verfahren, die nicht Gegenstand der Ansprüche sind, zum Herstellen einer Abbildungseinrichtung sind insbesondere geeignet zum Herstellen einer Abbildungseinrichtung für ein Endoskop und können Teil eines Verfahrens zum Herstellen eines Endoskops sein.

### Kurzbeschreibung der Figuren

Nachfolgend werden Ausführungsformen anhand der beigefügten Figuren näher erläutert. Es zeigen:
- Figur 1: eine schematische Darstellung eines Endoskops;
- Figur 2: eine schematische Darstellung eines distalen Endes eines Endoskops;
- Figur 3: eine schematische Darstellung eines distalen Endes eines weiteren Endoskops;
- Figur 4: eine schematische Darstellung eines distalen Endes eines weiteren Endoskops;
- Figur 5: eine schematische axonometrische Darstellung einer Abbildungseinrichtung;
- Figur 6: ein schematisches Flussdiagramm eines Verfahrens zum Herstellen einer Abbildungseinrichtung.

### Beschreibung der Ausführungsformen

Figur 1 zeigt eine schematische Darstellung eines Endoskops 10 mit einem proximalen Ende 11 und einem distalen Ende 12. Das Endoskop 10 weist eine in Figur 1 durch einen Pfeil angedeutete Blickrichtung 18 und ein Blickfeld oder Sichtfeld, dessen Ränder durch gestrichelte Linien angedeutet sind, auf.

Das Endoskop 10 weist einen langen und dünnen Schaft 20 mit einem proximalen Ende 21 nahe dem proximalen Ende 11 des Endoskops auf. Bei dem in Figur 1 dargestellten Beispiel ist der Querschnitt des Schafts 20 über die gesamte Länge des Schafts 20 konstant oder im Wesentlichen konstant. Abweichend von der Darstellung in Figur 1 kann der Querschnitt des Schafts variieren. Ein distales Ende 22 des Schafts 20 bildet das distale Ende 12 des Endoskops 10. Bei dem dargestellten Beispiel ist die Blickrichtung 18 des Endoskops 10 nicht parallel zu der Längsachse des Schafts 20 nahe dessen distalem Ende 22.

Bei dem dargestellten Beispiel ist der Schaft 20 flexibel. Das Endoskop 10 ist beispielsweise ein Sialendoskop zur optischen Inspektion eines Speichelgangs, ein Fetoskop zur optischen Inspektion eines Fötus in der Fruchtblase der Mutter, ein Zystoskop zur Blasenspiegelung. Alternativ kann das Endoskop 10 beispielsweise für die optische Inspektion eines Wurzelkanals eines Zahns in der Endodontie oder für die Duktoskopie, das heißt für die optische Inspektion eines Milchkanals einer Brustdrüse vorgesehen und ausgebildet sein.

Das Endoskop 10 weist an seinem distalen Ende 12 eine optische Abbildungseinrichtung zur Erzeugung eines reellen Bilds auf. Das Endoskop 10 kann ferner einen oder mehrere Bildsensoren an seinem distalen Ende 12 zum Erfassen des von der Abbildungseinrichtung erzeugten reellen Bilds aufweisen. Alternativ kann das Endoskop 10 eine Bildübertragungseinrichtung zum Weiterleiten des von der Abbildungseinrichtung erzeugten reellen Bilds zu dem proximalen Ende 11 des Endoskops aufweisen. In diesem Fall kann das zu dem proximalen Ende 11 weitergeleitete reelle Bild durch ein Okular, wie es in Figur 1 angedeutet ist, betrachtet und/oder mittels eines oder mehrerer Bildsensoren erfasst werden, die ein Bildsignal erzeugen. Ein oder mehrere Bildsensoren können in das Endoskop 10 nahe dessen proximalem Ende 11 integriert oder Bestandteil einer Kamera, die mit dem Okular des Endoskops optisch und mechanisch koppelbar ist, sein.

Figur 2 zeigt eine schematische Darstellung eines Schnitts durch ein distales Ende 12 eines Endoskops, das in einigen Merkmalen, Eigenschaften und Funktionen dem anhand der Figur 1 dargestellten Endoskop ähneln kann. Die Schnittebene der Figur 2 enthält eine Längsachse 28 des distalen Endes 22 des Schafts 20, die in Figur 2 als Symmetrieachse der äußeren Oberfläche eines Mantelbauteils 24 des Schafts 20 dargestellt ist. Schnittflächen optisch nicht transparenter Bauteile sind in Figur 2 schraffiert dargestellt. Schnittflächen optisch transparenter Bauteile sind in Figur 2 ohne Schraffur dargestellt.

Die Blickrichtung 18 des Endoskops 10 schließt mit der Längsachse 28 des Schafts 20 und mit deren Parallele 28' einen Winkel α (alpha) ein. Das Sichtfeld oder Blickfeld des Endoskops umfasst einen Winkel β (beta).

An dem distalen Ende 22 des Schafts 20 ist eine Öffnung 25 in dem optisch nicht transparenten Mantelbauteil 24 vorgesehen. Ein erster optisch transparenter Körper 32 verschließt die Öffnung 25 in dem Mantelbauteil 24 insbesondere fluiddicht oder hermetisch. Eine Lichteintrittsfläche 31 des ersten transparenten Körpers 32 bildet eine Lichteintrittsfläche des Endoskops. Bei dem in Figur 2 dargestellten Beispiel ist die Lichteintrittsfläche 31 des ersten transparenten Körpers 32 bündig mit der äußeren Oberfläche des Mantelbauteils 24 des Schafts 20 angeordnet. Der erste transparente Körper 32 weist eine Lichtaustrittsfläche 33 auf. Bei dem dargestellten Beispiel sind sowohl die Lichteintrittsfläche 31 als auch die Lichtaustrittsfläche 33 des ersten transparenten Körpers 32 gekrümmt.

An dem distalen Ende 22 des Schafts 20 und innerhalb des Mantelbauteils 24 ist ferner ein zweiter optisch transparenter Körper 35 mit einer Lichteintrittsfläche 34 und einer Lichtaustrittsfläche 36 angeordnet. Der zweite transparente Körper 35 kann unmittelbar auf dem Bildsensor 60 gedruckt oder auf andere Weise unmittelbar an der Lichteintrittsfläche 63 des Bildsensors 60 erzeugt und dabei stoffschlüssig mit ihm verbunden sein. Alternativ kann der zweite transparente Körper 35 zunächst separat hergestellt sein, wonach seine Lichtaustrittsfläche 36 mit einer Lichteintrittsfläche 63 des Bildsensors 60 gefügt wird.

Der zweite transparente Körper 35 kann wie in Figur 2 angedeutet eine Ausnehmung aufweisen, in die der Bildsensor 60 eingesetzt ist. Formschluss zwischen der Ausnehmung und dem Bildsensor 60 kann eine präzise Ausrichtung des Bildsensors 60 relativ zu dem zweiten transparenten Körper 35 auch im Fall einer zunächst separaten Herstellung ermöglichen.

Mehrere Stützstreben 39 verbinden den ersten transparenten Körper 32 und den zweiten transparenten Körper 35 mechanisch starr. Die Enden der Stützstreben 39 sind mit Randbereichen des ersten transparenten Körpers 32 und des zweiten transparenten Körpers 35 verbunden. Bei dem dargestellten Beispiel liegen die Stützstreben 39 außerhalb der in Figur 2 dargestellten Schnittebene. Deshalb sind die Konturen der Stützstreben 39 in punktierten Linien angedeutet.

Die transparenten Körper 32, 35 und die Stützstreben 39 sind insbesondere gleichzeitig und aus dem gleichen optisch transparenten Material gebildet. Die optisch transparenten Körper 32, 35 und optional auch die Stützstreben 39 sind insbesondere mittels eines auf Mehrphotonen-Absorption (insbesondere Zwei-Photonen-Absorption) beruhenden 3D-Druckverfahrens gebildet. Alternativ und abweichend von den Gegenständen der Ansprüche kann einer der transparenten Körper 32, 35 oder beide transparente Körper 32, 35 durch Abguss einer Gussform hergestellt werden, wobei die Gussform insbesondere durch einen 3D-Druck, der auf Mehrphotonen-Absorption (insbesondere Zwei-Photonen-Absorption) beruhen kann, hergestellt ist.

Alternativ können die transparenten Körper 32, 35 aus unterschiedlichen Materialien gebildet sein. Jeder der beiden transparenten Körper 32, 35 kann alternativ aus mehreren Teilkörpern aus unterschiedlichen Materialien gebildet sein, um beispielsweise eine chromatische Aberration zu reduzieren.

Nahe dem distalen Ende 22 des Schafts 20 ist ferner ein Bildsensor 60 innerhalb des Mantelbauteils 24 angeordnet. Eine Lichteintrittsfläche 63 des Bildsensors 60 liegt flächig an der ebenen Lichtaustrittsfläche 36 des zweiten transparenten Körpers 35 an und ist mit dieser insbesondere stoffschlüssig verbunden. Der Bildsensor 60 weist eine Vielzahl kleiner lichtempfindlicher Bereiche, die als Bildpunkte oder Pixel bezeichnet werden, in einer beispielsweise matrixförmigen Anordnung auf. Diese Bildpunkte oder Pixel sind in einer dünnen Schicht nahe der Lichteintrittsfläche 63 des Bildsensors 60 angeordnet. Vereinfachend wird hier angenommen, dass die Bildpunkte oder Pixel unmittelbar an der Lichteintrittsfläche 63 des Bildsensors 60 angeordnet sind.

Ein in der matrixförmigen Anordnung von Bildpunkten oder Pixeln erzeugtes Bild wird von dem Bildsensor 60 erfasst. Der Bildsensor 60 erzeugt ein analoges oder digitales und insbesondere elektrisches Bildsignal, das das erfasste Bild repräsentiert.

Von einem Objekt außerhalb des Endoskops ausgehendes Licht kann durch die Lichteintrittsfläche 31 des ersten transparenten Körpers 32 in das distale Ende 12 des Endoskops eintreten, durch die Lichtaustrittsfläche 33 des ersten transparenten Körpers 32 aus dem ersten transparenten Körper 32 austreten, durch die Lichteintrittsfläche 34 des zweiten transparenten Körpers 35 in diesen eintreten, durch die Lichtaustrittsfläche 36 des zweiten transparenten Körpers 35 aus diesem austreten und gleichzeitig durch die Lichteintrittsfläche 63 des Bildsensors 60 in diesen eintreten. Die Lichteintrittsfläche 31 und die Lichtaustrittsfläche 33 des ersten transparenten Körpers 32 und die Lichteintrittsfläche 34 des zweiten transparenten Körpers 35 sind jeweils gekrümmt. In Figur 2 sind insbesondere die Lichtaustrittsfläche 33 des ersten transparenten Körpers 32 und die Lichteintrittsfläche 34 des zweiten transparenten Körpers 35 jeweils als asphärisch (d. h. nicht-sphärisch) gekrümmt angedeutet.

Der erste transparenten Körper 32 weist einen Brechungsindex auf, der sich von dem Brechungsindex des Mediums (insbesondere Luft oder Kohlenstoffdioxid oder ein anderes Gas oder Wasser oder eine wässrige Lösung), in dem das distale Ende 22 des Schafts 20 sich bei der vorgesehenen Verwendung des Endoskops befindet, unterscheidet. Deshalb ist die Lichteintrittsfläche 31 des ersten transparenten Körpers 32 eine lichtbrechende Grenzfläche. Zwischen den transparenten Körpern 32, 35 befindet sich ein Medium (beispielsweise Luft oder Stickstoff), dessen Brechungsindex sich von den Brechungsindizes der Materialien der transparenten Körper 32, 35 unterscheidet. Deshalb sind auch die Lichtaustrittsfläche 33 des ersten transparenten Körpers 32 und die Lichteintrittsfläche 34 des zweiten transparenten Körpers 35 lichtbrechende Grenzflächen.

Sofern die Brechungsindizes der Materialien des zweiten transparenten Körpers 35 und des Bildsensors 60 sich unterscheiden, ist auch die durch die Lichtaustrittsfläche 36 des zweiten transparenten Körpers 35 und die Lichteintrittsfläche 63 des Bildsensors 60 gebildete Grenzfläche lichtbrechend. Die lichtbrechende Eigenschaft dieser Grenzfläche 36, 63 zwischen dem zweiten transparenten Körper 35 und dem Bildsensor 60 spielt aber nur insoweit eine Rolle als die als Bildpunkte oder Pixel bezeichneten lichtempfindlichen Bereiche des Bildsensors 60, die zur Erzeugung eines Bildsignals verwendet werden, von der Lichteintrittsfläche 63 des Bildsensors 60 beabstandet sind. Wie erwähnt wird hier vereinfachend davon ausgegangen, dass diese lichtempfindlichen Bereiche des Bildsensors 60 dünn und unmittelbar an der Lichteintrittsfläche 63 des Bildsensors 60 angeordnet sind.

Jede der lichtbrechenden Grenzflächen 31, 33, 34 ist gekrümmt. Jede der lichtbrechenden Grenzflächen 31, 33, 34 ist insbesondere asphärisch gekrümmt. Dies ist in Figur 2 insbesondere bei der Lichtaustrittsfläche 33 des ersten transparenten Körpers 32 erkennbar angedeutet. Jede der lichtbrechenden Grenzflächen 31, 33, 34 ist insbesondere auch nicht rotationssymmetrisch. Dies ist bei der Darstellung in Figur 2 insbesondere bei den lichtbrechenden Grenzflächen 33, 34 erkennbar angedeutet. Jede der lichtbrechenden Grenzflächen 31, 33, 34 ist insbesondere ferner auch kein Ausschnitt aus einer rotationssymmetrischen Fläche. Jede der lichtbrechenden Grenzflächen 31, 33, 34 ist insbesondere als Freiformfläche ausgebildet und beispielsweise zumindest stückweise durch eine oder mehrere polynomiale Funktionen beschreibbar.

Zumindest ein Teil der lichtbrechenden Grenzflächen 31, 33, 34 (bei dem in Figur 2 dargestellten Beispiel: alle lichtbrechenden Grenzflächen 31, 33, 34) sind gegeneinander und/ oder gegenüber der Längsachse 28 des distalen Endes 22 des Schafts 20 und der Flächennormale der Lichteintrittsfläche 63 des Bildsensors 60 gekippt. Dies bedeutet insbesondere, dass die Flächennormalen der lichtbrechenden Grenzflächen 31, 33, 34 an deren Flächenmittelpunkten oder an deren Scheiteln oder an deren Punkten mit maximaler Krümmung oder die jeweils über die Grenzflächen gemittelten Flächennormalen der lichtbrechenden Grenzflächen nicht parallel zueinander und/oder nicht parallel zu der Längsachse 28 des Schafts 20 oder zu der Flächennormale der Lichteintrittsfläche 63 des Bildsensors 60 sind.

Von Objekten außerhalb des distalen Endes 22 des Schafts 20 ausgehendes Licht kann durch die Lichteintrittsfläche 31 in den Schaft 20 eintreten und von der Lichteintrittsfläche 31 und den weiteren lichtbrechenden Grenzflächen 33, 34 gebrochen werden. Der erste transparente Körper 32 und der zweite transparente Körper 35 bilden eine optische Abbildungseinrichtung, die von Gegenständen innerhalb einer vorgesehenen Gegenstandsfläche ein scharfes Bild in den Bildpunkten oder Pixeln des Bildsensors 60 an dessen Lichteintrittsfläche 63 erzeugt. Dies ist in Figur 2 durch gestrichelte Linien angedeutet.

Bei dem dargestellten Beispiel bewirken die gekrümmten, zumindest teilweise weder sphärisch noch anders rotationssymmetrisch gekrümmten und gegeneinander gekippten lichtbrechenden Grenzflächen 31, 33, 34, dass die Blickrichtung 18 nicht parallel zu der Längsachse 28 des Schafts 20 an dessen distalem Ende 22 und nicht parallel zu der Flächennormale der Lichteintrittsfläche 63 des Bildsensors 60 ist, dass die vorbestimmten Gegenstandsfläche, die scharf abgebildet wird, eben oder im Wesentlichen eben ist, und dass die vorbestimmte Gegenstandsfläche, die scharf abgebildet wird, orthogonal oder im Wesentlichen orthogonal zu der Längsachse 28 des Schafts 20 an dessen distalem Ende 22 ist. Ferner kann die zumindest teilweise asphärische und nicht rotationssymmetrische Ausgestaltung und gegeneinander gekippte Anordnung der lichtbrechenden Grenzflächen 31, 33, 34 eine Verzerrung des erzeugten Bilds, also eine Variation des Abbildungsmaßstabs innerhalb des Bilds und abhängig von der Richtung, bewirken.

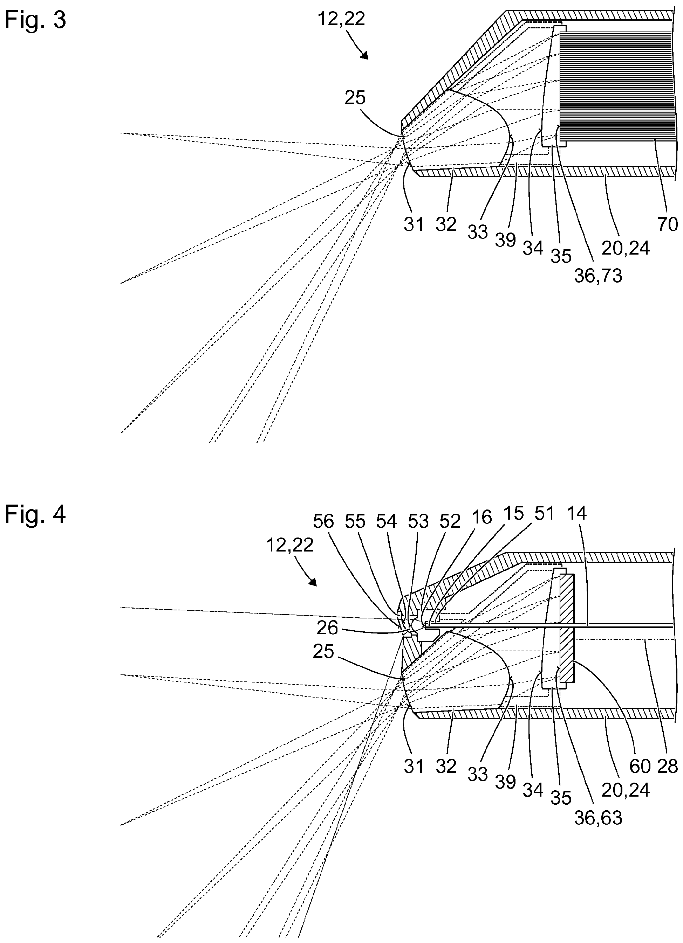

Figur 3 zeigt eine schematische Darstellung eines Schnitts durch das distale Ende 12 eines weiteren Endoskops, das in einigen Merkmalen, Eigenschaften und Funktionen den anhand der Figuren 1 und 2 dargestellten Endoskopen ähnelt. Die Art der Darstellung, insbesondere die Position und Orientierung der Schnittebene entspricht derjenigen der Figur 2. Nachfolgend sind insbesondere Merkmale, Eigenschaften und Funktionen beschrieben, in denen das Endoskop, dessen distales Ende 12 in Figur 3 dargestellt ist, sich von den anhand der Figuren 1 und 2 dargestellten Endoskopen unterscheidet.

Das Endoskop, dessen distales Ende in Figur 3 dargestellt ist, unterscheidet sich von dem anhand der Figur 2 dargestellten Endoskop insbesondere dadurch, dass kein Bildsensor am distalen Ende 22 des Schafts 20 vorgesehen ist. Stattdessen weist das Endoskop eine Bildübertragungseinrichtung 70 auf, die in Figur 3 beispielhaft als geordnetes Bündel von Lichtleitfasern dargestellt ist.

Eine Lichteintrittsfläche 73 der Bildübertragungseinrichtung 70 liegt flächig an der Lichtaustrittsfläche 36 des zweiten transparenten Körpers 35 an und kann mit dieser flächig gefügt sein, beispielsweise durch eine Klebung oder Schweißung. Alternativ kann der zweite transparente Körper 35, beispielsweise durch 3D-Druck, unmittelbar an dem distalen Ende der Bildübertragungseinrichtung 70 hergestellt und dabei stoffschlüssig mit der Lichteintrittsfläche 73 der Bildübertragungseinrichtung 70 verbunden sein.

Figur 4 zeigt eine schematische Darstellung eines Schnitts durch ein distales Ende 12 eines weiteren Endoskops, das in einigen Merkmalen, Eigenschaften und Funktionen den anhand der Figuren 1 bis 3 dargestellten Endoskopen, insbesondere dem anhand der Figur 2 dargestellten Endoskop ähnelt. Die Art der Darstellung, insbesondere Position und Orientierung der Schnittebene, entspricht derjenigen der Figuren 2 und 3. Nachfolgend sind insbesondere Merkmale, Eigenschaften und Funktionen des Endoskops, dessen distales Ende 12 in Figur 4 dargestellt ist, in denen dieses sich von den anhand der Figuren 1 bis 3 dargestellten Endoskopen unterscheidet, dargestellt.

Das Endoskop, dessen distales Ende 12 in Figur 4 dargestellt ist, weist einen Lichtleiter 14 auf, der sich in dem Schaft 20 des Endoskops von dessen proximalem Ende bis zu dessen distalem Ende 12 erstreckt. Das distale Ende 15 des Lichtleiters 14 ist in einer Bohrung 51 eines ersten transparenten Körpers 52 einer Lichtlenkeinrichtung angeordnet. Die Lichtlenkeinrichtung umfasst neben dem ersten transparenten Körper 52 einen zweiten transparenten Körper 55, der eine zweite Öffnung 26 in dem Mantelbauteil 24 fluiddicht oder hermetisch dicht verschließt.

Der erste transparente Körper 52 der Lichtlenkeinrichtung weist eine gekrümmte Lichtaustrittsfläche 53 auf, die an einer von der Ausnehmung 51 abgewandten Seite des ersten transparenten Körpers 52 der Lichtlenkeinrichtung angeordnet ist. Die Ausnehmung 51 definiert formschlüssig die Position und Orientierung des distalen Endes 15 des Lichtleiters 14 und damit auch einer Lichtaustrittsfläche 16 des Lichtleiters 14 relativ zu der Lichtaustrittsfläche 53 des ersten transparenten Körpers 52 der Lichtlenkeinrichtung. Das distale Ende 15 des Lichtleiters 14 ist in der Ausnehmung 51 des ersten transparenten Körpers 52 der Lichtlenkeinrichtung beispielsweise durch eine Klebung oder eine Schweißung stoffschlüssig befestigt.

Der zweite transparente Körper 55 der Lichtlenkeinrichtung weist eine der Lichtaustrittsfläche 53 des ersten transparenten Körpers 52 zugewandte Lichteintrittsfläche 54 und an einer von der Lichteintrittsfläche 54 abgewandten Seite eine Lichtaustrittsfläche 56 auf. Die Lichtaustrittsfläche 56 des zweiten transparenten Körpers 55 ist Teil der äußeren Oberfläche des distalen Endes 22 des Schafts 20 des Endoskops. Bei dem dargestellten Beispiel ist die Lichtaustrittsfläche 56 des zweiten transparenten Körpers 55 der Lichtlenkeinrichtung bündig mit umgebenden Bereichen der äußeren Oberfläche des Mantelbauteils 24 angeordnet.

Der erste transparente Körper 52 und der zweite transparente Körper 55 der Lichtlenkeinrichtung können das gleiche oder unterschiedliche Materialien mit unterschiedlichen Brechungsindizes aufweisen. Die Brechungsindizes der Materialien der transparenten Körper 52, 55 der Lichtlenkeinrichtung unterscheiden sich von dem Brechungsindex des Gases (beispielsweise Luft oder Stickstoff) in dem Zwischenraum zwischen den transparenten Körpern 52, 55 in der zweiten Öffnung 26 in dem Mantelbauteil 24. Deshalb sind die Lichtaustrittsfläche 53 des ersten transparenten Körpers und die Lichteintrittsfläche 54 des zweiten transparenten Körpers 55 der Lichtlenkeinrichtung lichtbrechende Grenzflächen. Der zweite transparenten Körper 55 der Lichtlenkeinrichtung weist einen Brechungsindex auf, der sich von dem Brechungsindex des Mediums (insbesondere Luft oder Kohlenstoffdioxid oder ein anderes Gas oder Wasser oder eine wässrige Lösung), in dem das distale Ende 22 des Schafts 20 sich bei der vorgesehenen Verwendung des Endoskops befindet, unterscheidet. Deshalb ist auch die Lichtaustrittsfläche 56 des zweiten transparenten Körpers 55 der Lichtlenkeinrichtung eine lichtbrechende Grenzfläche.

Bei dem dargestellten Beispiel ist die Lichteintrittsfläche 54 des zweiten transparenten Körpers 55 der Lichtlenkeinrichtung gekrümmt, die Lichtaustrittsfläche 56 des zweiten transparenten Körpers 55 ist eben. Insbesondere ist mindestens eine der lichtbrechenden Grenzflächen 53, 54, 56 asphärisch, nicht rotationssymmetrisch oder nicht Ausschnitt aus einer rotationssymmetrischen Fläche.

Bei dem dargestellten Beispiel sind die lichtbrechenden Grenzflächen 53, 54, 56 ferner gegeneinander gekippt in dem Sinn, der oben für die lichtbrechenden Grenzflächen 31, 33, 34 der optischen Abbildungseinrichtung 32, 35 beschrieben ist.

Die transparenten Körper 52, 55 der Lichtlenkeinrichtung steuern mit ihren gekrümmten und gegeneinander gekippten lichtbrechenden Grenzflächen 53, 54, 56 die Verteilung von Beleuchtungslicht, das von dem Lichtleiter 14 zu dem distalen Ende 12 des Endoskops übertragen wird und dort durch die transparenten Körper 52, 55 der Lichtlenkeinrichtung hindurchtritt und aus dem distalen Ende 22 des Schafts 20 austritt. Die Verteilung des Beleuchtungslichts ist insbesondere an das Sichtfeld oder Blickfeld des Endoskops angepasst.

Der von dem distalen Ende 15 des Lichtleiters 14 ausgehende Lichtstrom und die Grenzen des ausgeleuchteten Bereichs sind in Figur 4 durch punktierte dünne Linien angedeutet.

Der Lichtleiter 14 kann Beleuchtungslicht, das von einer Lichtquelle in dem proximalen Ende des Endoskops erzeugt oder mittels eines Lichtleitkabels von einer externen Lichtquelle zu dem proximalen Ende des Endoskops übertragen wird, zu dem distalen Ende 12 des Endoskops übertragen. Alternativ kann der Lichtleiter 14 Beleuchtungslicht, das von einer Lichtquelle in dem Schaft 20 nahe dessen distalem Ende erzeugt wird, zu der Lichtlenkeinrichtung 52, 55 übertragen. Alternativ und abweichend von der Darstellung in Figur 4 kann anstelle des Lichtleiters 14 eine Lichtquelle vorgesehen sein, die unmittelbar an der Lichtlenkeinrichtung 52, 55, beispielsweise in der Ausnehmung 51 oder an einer Lichteintrittsfläche des ersten transparenten Körpers 52 der Lichtlenkeinrichtung angeordnet ist.

Bei dem in Figur 4 dargestellten Beispiel weist das Endoskop einen Bildsensor 60 innerhalb des Mantelbauteils 24 nahe dem distalen Ende 22 des Schafts 20 auf, ähnlich wie dies bei dem anhand der Figur 2 dargestellten Beispiel der Fall ist. Alternativ kann anstelle des Bildsensors 60 eine Bildübertragungseinrichtung vorgesehen sein, beispielsweise ein geordnetes Bündel von Lichtleitfasern wie es anhand der Figur 3 dargestellt ist.

Figur 5 zeigt eine schematische axonometrische Darstellung einer optischen Abbildungseinrichtung 30 und eines Bildsensors 60, die in einigen Merkmalen, Eigenschaften und Funktionen den Abbildungseinrichtungen der anhand der Figuren 2 bis 4 dargestellten Endoskope ähneln. Die Abbildungseinrichtung 30 ist teilweise aufgeschnitten dargestellt, so dass Konturen von Schnittebenen entlang zweier orthogonaler Halbebenen sichtbar sind. Der Bildsensor 60 ist einfach axonometrisch ohne Schnitt und vereinfachend als Quader dargestellt.

Wie bei den anhand der Figuren 2 bis 4 dargestellten Beispielen bilden gekrümmte und gegeneinander gekippte Lichteintrittsflächen und Lichtaustrittsflächen 31, 33, 34 zweier transparenter Körper 32, 35 lichtbrechende Grenzflächen mit abbildender Wirkung. Eine Lichtaustrittsfläche 36 des zweiten transparenten Körpers 35 ist mit einer Lichteintrittsfläche 63 des Bildsensors 60 gefügt. Die Krümmung der lichtbrechenden Grenzflächen 31, 33, 34 und ihre gegeneinander und gegenüber der Lichteintrittsfläche 63 des Bildsensors 60 gekippte Anordnung schaffen eine Blickrichtung, die nicht orthogonal zu der Lichteintrittsfläche 63 des Bildsensors 60 ist.

An der Lichteintrittsfläche 31 des ersten transparenten Körpers 32 ist ein diffraktives optisches Element 40 aus einer Mehrzahl von ringförmigen (jedoch keinesfalls notwendigerweise kreisringförmigen) Stufen vorgesehen. Das diffraktive optische Element 40 weist eine anomale Dispersion auf und kann so die chromatische Aberration der optischen Abbildungseinrichtung 30 korrigieren oder vermindern.

Abweichend von der Darstellung in Figur 5 kann alternativ oder zusätzlich zu dem diffraktiven Element 40 an der Lichteintrittsfläche 31 des ersten transparenten Körpers 32 ein diffraktives optisches Element an der Lichtaustrittsfläche 33 des ersten transparenten Körpers 32 oder an der Lichteintrittsfläche 34 des zweiten transparenten Körpers 35 der optischen Abbildungseinrichtung 30 vorgesehen sein.

Der erste Körper 32 und der zweite Körper 35 der optischen Abbildungseinrichtung 30 sind durch mehrere parallele Stützstreben 39, die mit den transparenten Körpern 32, 35 eine Käfigform bilden, mechanisch starr verbunden. Die transparenten Körper 32, 35 und die Stützstreben 39 können aus dem gleichen Material gebildet oder insbesondere während des gleichen Herstellungsschritts - beispielsweise durch 3D-Druck - erzeugt werden.

Figur 6 zeigt ein schematisches Flussdiagramm eines Verfahrens, das nicht Gegenstand der Ansprüche ist, zum Herstellen einer Abbildungseinrichtung für ein Endoskop. Das Verfahren ist insbesondere zur Herstellung einer Abbildungseinrichtung mit den anhand der Figuren 2 bis 5 dargestellten Eigenschaften und/oder für ein Endoskop, wie es anhand der Figuren 1 bis 4 dargestellt ist, geeignet. Das Verfahren ist jedoch auch zur Herstellung einer Abbildungseinrichtung geeignet, die Merkmale Eigenschaften und Funktionen aufweist, die von den anhand der Figuren 1 bis 5 dargestellten abweichen. Nachfolgend sind beispielhaft Bezugszeichen aus den Figuren 1 bis 5 verwendet, um das Verständnis zu vereinfachen.

Bei einem ersten Schritt 101 wird mittels 3D-Druck eine Gussform hergestellt. Bei einem zweiten Schritt 102 wird durch Abguss der Gussform eine Abbildungseinrichtung hergestellt. Die Gussform kann als verlorene Form ausgebildet sein, die nach dem Abguss zerstört werden muss, um die Abbildungseinrichtung als Gussteil vollständig freizulegen. Alternativ kann die Gussform wieder verwendbar sein.

### Bezugszeichen

- 10: **Endoskop**
- 11: proximales Ende des Endoskops 10
- 12: distales Ende des Endoskops 10
- 14: Lichtleiter zum Übertragen von Beleuchtungslicht
- 15: distales Ende des Lichtleiters 14
- 16: Lichtaustrittsfläche des Lichtleiters 14
- 18: Blickrichtung des Endoskops 10
- 20: **Schaft** des Endoskops 10
- 21: proximales Ende des Schafts 20
- 22: distales Ende des Schafts 20
- 24: Mantelbauteil des Schafts 20
- 25: erste Öffnung in dem Mantelbauteil 24, zur Aufnahme des ersten transparenten Körpers 32 der optischen Abbildungseinrichtung 30
- 26: zweite Öffnung in dem Mantelbauteil 24, zur Aufnahme des ersten transparenten Körpers 52 einer Lichtlenkeinrichtung
- 28: Längsachse des Schafts 20 oder des distalen Endes 23 des Schafts 20
- 28': Parallele der Längsachse 28
- 30: **optische Abbildungseinrichtung** an dem distalen Ende 23 des Schafts 20
- 31: Lichteintrittsfläche des ersten transparenten Körpers 32, der optischen Abbildungseinrichtung 30 und des Endoskops 10
- 32: **erster transparenter Körper** der optischen Abbildungseinrichtung 30
- 33: Lichtaustrittsfläche des ersten transparenten Körpers 32 der optischen Abbildungseinrichtung 30
- 34: Lichteintrittsfläche des zweiten transparenten Körpers 35 der optischen Abbildungseinrichtung 30
- 35: **zweiter transparenter Körper** der optischen Abbildungseinrichtung 30
- 36: Lichtaustrittsfläche des zweiten transparenten Körpers 35 der optischen Abbildungseinrichtung 30
- 39: Stützstrebe zur starren mechanischen Verbindung des ersten transparenten Körpers 32 und des zweiten transparenten Körpers 35 der optischen Abbildungseinrichtung 30
- 40: stufenförmige Struktur an der Lichteintrittsfläche 31 des ersten transparenten Körpers 32 als diffraktives optisches Element
- 51: Ausnehmung in dem ersten transparenten Körper 52 einer Lichtlenkeinrichtung zum Aufnehmen des distalen Endes des Lichtleiters 15
- 52: **erster transparenter Körper** der Lichtlenkeinrichtung
- 53: Lichtaustrittsfläche des ersten transparenten Körpers 52 der Lichtlenkeinrichtung
- 54: Lichteintrittsfläche des zweiten transparenten Körpers 55 der Lichtlenkeinrichtung
- 55: **zweiter transparenter Körper** der Lichtlenkeinrichtung
- 56: Lichtaustrittsfläche des zweiten transparenten Körpers 55 der Lichtlenkeinrichtung
- 60: Bildsensor
- 63: Lichteintrittsfläche des Bildsensors 60
- 70: Bildübertragungseinrichtung
- 73: Lichteintrittsfläche der Bildübertragungseinrichtung 70
- 101: erster Schritt (Herstellen einer Gussform mittels 3D-Druck)
- 102: zweiter Schritt (Herstellen einer Abbildungseinrichtung durch Abguss der Gussform)

## Patentansprüche

1. **Medizinisches Endoskop** (10) mit:
einem **Schaft** (20) mit einem distalen Ende (23);
einer optischen **Abbildungseinrichtung** (30) an dem distalen Ende (23) des Schafts (20) eingerichtet zum Erzeugen eines reellen Bilds eines mittels des medizinischen Endoskops (10) betrachteten Objekts;
zumindest entweder einer Bildübertragungseinrichtung (70) eingerichtet zum Weiterleiten des reellen Bilds oder einem **Bildsensor** (60) eingerichtet zum Erfassen des reellen Bilds,
wobei die Abbildungseinrichtung (30) **gekrümmte lichtbrechende Grenzflächen** (31, 33, 34) aufweist, die gegeneinander **gekippt** sind,
wobei die **Blickrichtung** (18) des medizinischen Endoskops (10) **nicht parallel** zu der Längsachse (28) des distalen Endes (22) des Schafts (20) des medizinischen Endoskops (10) ist,
**dadurch gekennzeichnet, dass**
die Abbildungseinrichtung (30) mittels **3D-Druck** hergestellt ist, und
durch eine **Lichtlenkeinrichtung** (52, 55) an dem distalen Ende (22) des medizinischen Endoskops (20) eingerichtet zum **Lenken von** aus einer distalen Lichtaustrittsfläche (16) eines Lichtleiters (14) oder aus einer Lichtaustrittsfläche einer Lichtquelle austretendem **Beleuchtungslicht,** und
dadurch, dass die Lichtlenkeinrichtung (52, 55) eine lichtbrechende **Grenzfläche** (53, 54, 56), die gegenüber der Längsachse des distalen Endes (15) des Lichtleiters (14) oder gegenüber einer Flächennormalen der Lichtaustrittsfläche der Lichtquelle gekippt ist, aufweist, und
dadurch, dass die Abbildungseinrichtung (30) und die Lichtlenkeinrichtung (52, 55) **monolithisch** ausgebildet sind.

2. Medizinisches Endoskop (10) nach einem der vorangehenden Ansprüche, bei dem eine der gekrümmten lichtbrechenden Grenzflächen (31, 33, 34) der Abbildungseinrichtung (30) **kein** Ausschnitt aus einer **rotationssymmetrischen** gekrümmten Fläche ist.

3. Medizinisches Endoskop (10) nach einem der vorangehenden Ansprüche, wobei das medizinische Endoskop (10) keine reflektierende Fläche lichtstromaufwärts des von der Abbildungseinrichtung erzeugten reellen Bilds aufweist.

4. Medizinisches Endoskop (10) nach einem der vorangehenden Ansprüche, ferner mit:
einer Blendenstruktur zum lateralen Beschneiden eines optischen Pfads, wobei die Blendenstruktur zumindest entweder gleichzeitig mit der Abbildungseinrichtung (30) hergestellt oder in die Abbildungseinrichtung (30) eingebettet ist

5. Medizinisches Endoskop (10) nach einem der vorangehenden Ansprüche, bei dem die Abbildungseinrichtung (30) mittels auf Mehrphotonen-Absorption oder Mehrphotonen-Polymerisation beruhendem 3D-Druck hergestellt ist.

6. Medizinisches Endoskop (10) nach einem der vorangehenden Ansprüche, bei dem
die Abbildungseinrichtung (30) **unmittelbar auf** eine distale Lichteintrittsfläche (73) einer Bildübertragungseinrichtung (70) zum Weiterleiten des reellen Bilds oder auf eine Lichteintrittsfläche (63) eines **Bildsensors** (60) zum Erfassen des reellen Bilds **gedruckt** ist.

7. Medizinisches Endoskop (10) nach einem der vorangehenden Ansprüche, bei dem die Abbildungseinrichtung (30) mittels 3D-Druck aus mehreren **verschiedenen Materialien** hergestellt ist.

8. Medizinisches Endoskop (10) nach einem der vorangehenden Ansprüche, bei dem die Abbildungseinrichtung (30) eine als **diffraktives optisches Element** (40) ausgebildete Grenzfläche (31) oder ein anderes diffraktives optisches Element aufweist.

9. Medizinisches Endoskop (10) nach einem der vorangehenden Ansprüche, bei dem
die Abbildungseinrichtung (30) insbesondere **mehrere** optisch **transparente** und bei der vorgesehenen Verwendung von Licht, das von einem betrachteten Objekt ausgeht und zur Erzeugung des reellen Bild beiträgt, durchsetzte **Körper** (32, 35) mit den lichtbrechenden Grenzflächen (31, 33, 34, 36) aufweist,
eine **Stützeinrichtung** (39) die transparenten Körper (32, 35) mechanisch starr verbindet,
die Stützeinrichtung (39) und die transparenten Körper (32, 35) zumindest entweder aus dem **gleichen Material** gebildet oder **gleichzeitig** hergestellt sind.

10. Medizinisches Endoskop (10) nach einem der vorangehenden Ansprüche, ferner mit:
einem **Stützgerüst** (39), das die Abbildungseinrichtung (30) und die Lichtlenkeinrichtung (52, 55) miteinander starr verbindet.

11. Medizinisches Endoskop (10) nach einem der vorangehenden Ansprüche, bei dem
die Lichtlenkeinrichtung (52, 55) eine Ausnehmung (51) zur Aufnahme des distalen Endes (15) eines Lichtleiters (14) aufweist,
die Ausnehmung (51) die Position und die Orientierung des distalen Endes (15) des Lichtleiters (14) definiert.

12. Medizinisches Endoskop (10) nach einem der vorangehenden Ansprüche, bei dem die lichtbrechende Grenzfläche (53, 54, 56) der Lichtlenkeinrichtung (52, 55) nicht rotationssymmetrisch ist.

## Claims

1. **A medical endoscope** (10), comprising:
a **shaft** (20) comprising a distal end (23);
an optical **imaging means** (30) at the distal end (23) of the shaft (20) configured for producing a real image of an object observed by means of the medical endoscope (10);
at least one of an image transmission means (70) configured for transmitting the real image and an **image sensor** (60) for capturing the real image,
wherein the imaging means (30) has **curved light-refracting interfaces** (31, 33, 34), which are tilted relative to one another,
wherein the **viewing direction** (18) of the endoscope (10) is **not parallel** to the longitudinal axis (28) of the distal end (22) of the shaft (20) of the medical endoscope (10),
**characterized in that**
the imaging means (30) is produced by means of **3D printing,** and
by a **light-steering means** (52, 55) at the distal end (22) of the medical endoscope (20) configured for **steering illumination light** emanating from a distal light-exit surface (16) of a light guide (14) or from a light-exit surface of a light source, and
**in that** the light-steering means (52, 55) has a light-refracting interface (53, 54, 56) tilted relative to a longitudinal axis of the distal end (15) of the light guide (14) or relative to a surface normal of the light-exit surface of the light source, and
**in that** the imaging means (30) and the light steering means (52, 55) are embodied monolithically.

2. Medical endoscope (10) according to one of the preceding claims, wherein one of the curved light-refracting interfaces (31, 33, 34) of the imaging means (30) is not a part of a **rotationally symmetric** curved surface.

3. Medical endoscope (10) according to one of the preceding claims, wherein the medical endoscope (10) has no reflecting interface upstream of the real image produced by the imaging means.

4. Medical endoscope (10) according to one of the preceding claims, further comprising:
an aperture structure for laterally trimming an optical path, wherein the aperture structure is at least one of manufactured simultaneously with the imaging means (30) and embedded in the imaging means (30).

5. Medical endoscope (10) according to one of the preceding claims, wherein the imaging means (30) is produced by means of 3D printing based on multi-photon absorption or multi-photon polymerization..

6. Medical endoscope (10) according to one of the preceding claims, wherein
the imaging means (30) is **printed directly on** a distal light entrance surface (73) of an image transmission means (70) for transmitting the real image or on a light-entrance surface (63) of an **image sensor** (60) for capturing the real image.

7. Medical endoscope (10) according to one of the preceding claims, wherein the imaging means (30) is produced from a plurality of **different materials** by means of 3D printing.

8. Medical endoscope (10) according to one of the preceding claims, wherein the imaging means (30) comprises an interface (31) embodied as a **diffractive optical element** (40) or an other diffractive optical element.

9. Medical endoscope (10) according to one of the preceding claims, wherein
the imaging means (30) in particular comprises a **plurality** of optically **transparent bodies** (32, 35) providing the light-refracting interfaces (31, 33, 34, 36) and, in the intended use, penetrated by light emanating from an observed object and contributing to the production of the real image,
a **supporting means** (39) mechanically rigidly connects the bodies (32, 35),
the supporting means (39) and the transparent bodies (32, 35) are at least one of formed by the **same material** and produced **at the same time.**

10. Medical endoscope (10) according to one of the preceding claims, further comprising:
a **support frame** (39) rigidly connecting the imaging means (30) and the light-steering means (52, 55) to one another.

11. Medical endoscope (10) according to one of the preceding claims, wherein
the light-steering means (52, 55) has a cutout (51) for receiving the distal end (15) of the light guide (14),
the cutout (51) defines the position and the orientation of the distal end (15) of the light guide (14).

12. Medical endoscope (10) according to one of the preceding claims, wherein the light-refracting interface (53, 54, 56) of the light steering means (52, 55) is not rotationally symmetric.

## Revendications

1. **Endoscope médical** (10) comportant :
une **tige** (20) présentant une extrémité distale (23) ;
un **dispositif de formation d'image optique** (30) à l'extrémité distale (23) de la tige (20), conçu pour la production d'une image réelle d'un objet observé au moyen de l'endoscope médical (10) ;
au moins soit un dispositif de transmission d'image (70) conçu pour la transmission de l'image réelle, soit un **capteur d'image** (60) conçu pour la détection de l'image réelle,
le dispositif de formation d'image (30) présentant des **surfaces limites réfringentes incurvées** (31, 33, 34) qui sont inclinées les unes par rapport aux autres,
la **direction de vision** (18) de l'endoscope médical (10) **n'étant pas parallèle** à l'axe longitudinal (28) de l'extrémité distale (22) de la tige (20) de l'endoscope médical (10),
**caractérisé en ce que**
le dispositif de formation d'image (30) est produit par **impression 3D,** et
par un **dispositif de guidage de lumière** (52, 55) à l'extrémité distale (22) de l'endoscope médical (20), conçu pour le **guidage de lumière d'éclairage** sortant d'une surface distale (16) de sortie de lumière d'un guide de lumière (14) ou d'une surface de sortie de lumière d'une source de lumière, et
**en ce que** le dispositif de guidage de lumière (52, 55) présente une **surface limite réfringente** (53, 54, 56) qui est inclinée par rapport à l'axe longitudinal de l'extrémité distale (15) du guide de lumière (14) ou par rapport à une normale à la surface de la surface de sortie de lumière de la source de lumière, et
**en ce que** le dispositif de formation d'image (30) et le dispositif de guidage de lumière (52, 55) ont une configuration **monolithique.**

2. Endoscope médical (10) selon l'une quelconque des revendications précédentes, dans lequel l'une des surfaces limites réfringentes incurvées (31, 33, 34) du dispositif de formation d'image (30) **n'est** pas un segment d'une surface courbe à **symétrie de révolution.**

3. Endoscope médical (10) selon l'une quelconque des revendications précédentes, dans lequel l'endoscope médical (10) ne comporte pas de surface réfléchissante en amont, dans le sens du flux lumineux, de l'image réelle produite par le dispositif de formation d'image.

4. Endoscope médical (10) selon l'une quelconque des revendications précédentes, comportant en outre :
une structure de diaphragme destinée au rognage latéral d'un chemin optique, la structure de diaphragme étant au moins soit produite simultanément avec le dispositif de formation d'image (30), soit incorporée dans le dispositif de formation d'image (30).

5. Endoscope médical (10) selon l'une quelconque des revendications précédentes, dans lequel le dispositif de formation d'image (30) est produit par impression 3D basée sur l'absorption multiphotonique ou la polymérisation multiphotonique.

6. Endoscope médical (10) selon l'une quelconque des revendications précédentes, dans lequel
le dispositif de formation d'image (30) est **imprimé directement** sur une surface distale (73) d'entrée de lumière d'un dispositif de transmission d'image (70) destiné à la transmission de l'image réelle ou sur une surface d'entrée de lumière (63) d'un **capteur d'image** (60) destiné à la détection de l'image réelle.

7. Endoscope médical (10) selon l'une quelconque des revendications précédentes, dans lequel le dispositif de formation d'image (30) est produit par impression 3D à partir de **plusieurs matières différentes.**

8. Endoscope médical (10) selon l'une quelconque des revendications précédentes, dans lequel le dispositif de formation d'image (30) présente une surface limite (31) configurée en tant **qu'élément optique diffractif** (40) ou qu'un autre élément optique diffractif.

9. Endoscope médical (10) selon l'une quelconque des revendications précédentes, dans lequel
le dispositif de formation d'image (30) comporte en particulier **plusieurs corps** (32, 35) comportant les surfaces limites (31, 33, 34, 36) qui réfractent la lumière, optiquement **transparents** et traversés, dans le cas de l'utilisation prévue, par de la lumière qui provient d'un objet observé et contribue à la production de l'image réelle,
un **dispositif de support** (39) qui relie mécaniquement de façon rigide les corps transparents (32, 35),
le dispositif de support (39) et les corps transparents (32, 35) sont au moins soit formés à partir de la **même matière,** soit produits **simultanément.**

10. Endoscope médical (10) selon l'une quelconque des revendications précédentes, comportant en outre :
une **structure de support** (39) qui relie rigidement le dispositif de formation d'image (30) et le dispositif de guidage de lumière (52, 55).

11. Endoscope médical (10) selon l'une quelconque des revendications précédentes, dans lequel
le dispositif de guidage de lumière (52, 55) présente un évidement (51) destiné à la réception de l'extrémité distale (15) d'un guide de lumière (14),
l'évidement (51) définit la position et l'orientation de l'extrémité distale (15) du guide de lumière (14).

12. Endoscope médical (10) selon l'une quelconque des revendications précédentes, dans lequel la surface limite réfringente (53, 54, 56) du dispositif de guidage de lumière (52, 55) ne présente pas de symétrie de révolution.
